# EUROPÄISCHE PATENTSCHRIFT

(11) **EP 0 896 603 B1**
(45) Veröffentlichungstag und Bekanntmachung des Hinweises auf die Patenterteilung: **29.11.2000**
(21) Anmeldenummer: 97921742.9
(22) Anmeldetag: 24.04.1997
(51) Int. Cl.: C08J 3/12

(54) **IN WÄSSRIGER LÖSUNG REDISPERGIERBARE POLYMERPULVER**
POLYMER POWDERS REDISPERSIBLE IN AQUEOUS SOLUTION
POUDRES POLYMERES REDISPERSIBLES EN SOLUTION AQUEUSE

(30) Priorität: 03.05.1996 DE 19617716
(43) Veröffentlichungstag der Anmeldung: 17.02.1999
(73) Patentinhaber: BASF AKTIENGESELLSCHAFT, 67056 Ludwigshafen (DE)
(72) Erfinder: BREITENBACH, Jörg, D-68199 Mannheim (DE); KOLTER, Karl, D-67117 Limburgerhof (DE); SCHMITT, Angelika, D-67551 Worms (DE)
(86) Internationale Anmeldenummer: EP9702097
(87) Internationale Veröffentlichungsnummer: WO9742255

(56) Entgegenhaltungen:
- EP-A- 0 134 451
- EP-A- 0 274 053
- EP-A- 0 536 595
- EP-A- 0 601 518
- EP-A- 0 605 933
- WO-A-93/13048
- DE-A- 3 916 020
- DATABASE WPI Section Ch, Week 9125 Derwent Publications Ltd., London, GB; Class A14, AN 91-181465 XP002041664 & JP 03 109 409 A (NITTO DENKO CORP) , 9.Mai 1991

## Beschreibung

Die Erfindung betrifft ein Verfahren zur Herstellung von in wäßriger Lösung redispergierbare Polymerpulvern, enthaltend freie säure- oder basengruppentragende Copolymere und gegebenenfalls Hilfsstoffe, dadurch gekennzeichnet, daß man den pH-Wert der Dispersion durch Zugabe eines Puffersystems auf pH 2,0 bis 6,5 im Falle säuregruppentragender Copolymere bzw. pH 7,5 bis 12 im Falle basengruppentragender Copolymere einstellt.

Die Erfindung betrifft außerdem in wäßriger Lösung redispergierbare Polymerpulver sowie ihre Verwendung in kosmetischen oder pharmazeutischen Zubereitungen oder Zubereitungen für den Pflanzenschutz.

Das Patent DE 2 512 238 beschreibt filmbildende Polymerpulver, die aus wäßrigen Kunststoffdispersionen hergestellt werden. Diese filmbildenden Pulver werden nach Auflösen in einem organischen Lösungsmittel zum Überziehen fester Arzneiformen verwendet.

Die Verwendung organischer Lösungsmittel beim Überziehen fester Arzneiformen führt zu einem nicht unerheblichen technischen Aufwand. Alle benutzten Geräte müssen explosionsgeschützt ausgelegt sein, um eine Entzündung des Lösungsmittels zu verhindern. Aus Umwelt- sowie Kostengründen muß das abgedampfte Lösungsmittel aus der Abluft zurückgewonnen werden. Außerdem muß das Lösungsmittel rückstandsfrei aus den pharmazeutischen Zubereitungen entfernt werden. Es wurde deshalb nach alternativen zur Verwendung organischer Lösungsmittel gesucht.

In Folge wurden eine Reihe von Verfahren mit Wasser als Lösungsmittel entwickelt, die jedoch eine Reihe von Nachteilen besitzen.

So beschreibt Bright et al. im US-Patent 5 252 704 redispergierbare Polymerpulver, die aus Copolymerisaten durch Zusatz großer Mengen an Polyvinylpyrrolidon vor der Sprühtrocknung herstellbar sind. Der prozentual hohe Zusatz an Polyvinylpyrrolidon beeinflußt jedoch die Löslichkeit des nach der Verfilmung entstehenden Polymerfilms in unerwünschter Weise.

EP-B 88 951 und EP-B 161 326 beschreiben die Redispergierung von Emulsionspolymerisatpulver mit freien Carboxyl- oder Aminogruppen durch Überführen dieses Gruppen in ihre Salze. Dabei werden freie Carboxylgruppen durch Zusatz von Basen und freie Aminogruppen durch Zusatz von Säuren in ihre Salze überführt. Dies geschieht nach der Trocknung der Polymerdispersion durch Einrühren einer alkalischen Lösung in das in Wasser fein verteilte carboxylgruppentragende Polymerpulver.

Trägt das Polymer freie Aminogruppen, so wird eine saure Lösung zur Dispergierung eingerührt.

Im Falle carboxylgruppenhaltiger Polymere wird die Möglichkeit der teilweisen Neutralisation der freien Carboxylgruppen durch Alkalizugaben vor der Sprühtrocknung beschrieben.

Aus EP 601 518 ist es bekannt, carboxylgruppenhaltige Polymere vor der Sprühtrocknung durch Zugabe einer Base auf pH 7 zu neutralisieren.

DE 39 16 020 beschreibt vinpocetinhaltige Polylactidpartikel, die durch Einsatz eines basischen Puffersystems vor der Sprühtrocknung hergestellt wurden.

Dabei sind jedoch die Viskosität der Dispersion und die Koagulatbildung vor der Sprühtrocknung kritische Größen. Wird die Alkalizugabe vor der Trocknung durchgeführt, bilden sich außerdem bei der anschließenden Redispergierung leicht unerwünschte Koagulate. Es wird deshalb vorzugsweise die Neutralisation der Polymerpulver, d.h. die Salzbildung, nach Trocknung der Pulver direkt vor dem Herstellen der festen Arzneiformen vorgenommen. Für den Anwender stellt sich dadurch das Problem, zusätzliche Anlagen für die Neutralisation und Lagerung der Basen oder Säuren bereitzuhalten.

Es bestand daher die Aufgabe, redispergierbare Polymere, die sich ohne die beschriebenen Nachteile direkt zu wäßriger Lösung dispergieren lassen, zu entwickeln und für Anwendungen im kosmetischen und pharmazeutischen Bereich und Pflanzenschutz zur Verfügung zu stellen.

Demgemäß wurde nun gefunden, daß man den pH-Wert der Dispersion durch Zugabe mindestens eines Puffersystems auf pH 2,0 bis 6,5 im Falle säuregruppentragender Copolymere bzw. pH 7,5 bis 12 im Falle basengruppentragende Copolymere vor der Trocknung so einstellen kann, daß ein exzellent redispergierbares Polymerpulver herstellbar ist.

Das sehr lagerstabile erfindungsgemäße Polymerpulver redispergiert rasch in wäßriger Lösung ohne Bildung von zum Beispiel Koagulaten.

Sie sind daher ausgezeichnet als Filmbildner in kosmetischen oder pharmazeutischen Zubereitungen oder Zubereitungen für den Pflanzenschutz geeignet.

Für das erfindungsgemäße Verfahren eignen sich prinzipiell alle bekannten Puffer wie beispielsweise in Handbook of Biochemistry (Es. Sober, H.A., Horte R.A., The Chemical Rubber Co., 1968: J-195-J-199) beschrieben.

Der pH-Wert der freie säure- oder basengruppentragenden Copolymere enthaltenden Dispersion wird vorteilhafterweise durch Zugabe mindestens eines Puffersystems auf pH 2,0 bis 6,5 im Falle säuregruppentragender Copolymere bzw. pH 7,5 bis 12 im Falle basengruppentragender Copolymere vor der Trocknung eingestellt. Bevorzugt wird der pH-Wert auf pH 3,0 bis 6,0 im Falle säuregruppentragender Copolymere bzw. 7,5 bis 11 im Falle basengruppentragender Copolymere vor der Trocknung eingestellt. Durch die Einstellung des pH-Wertes wird ein Teil der freien Säure- oder Basengruppen der Copolymere in ihr Salz überführt.

Unter Puffersysteme sind übliche Puffer und/oder polymere Puffer zu verstehen.

Geeignete Puffer sind beispielsweise alle Salze aus schwachen Säuren und starken Basen oder starken Säuren und schwachen Basen, wobei es sich um Salze derselben Säuren oder Basen oder Mischungen aus unterschiedlichen Säuren oder Basen handeln kann.

Als Puffer bei freien säuregruppentragenden Copolymeren wie zum Beispiel bei Copolymeren mit freien Phosphono-, Sulfo- oder Carboxylgruppen wird mindestens ein Puffersystem verwendet. Bevorzugt wird dabei der Pufferbereich des Puffersystems zwischen pH 1 bis 7 gewählt. Als Puffer bzw. Pufferlösungen mit einem Pufferbereich im sauren Milieu zwischen pH 1 bis 7 kommen beispielsweise Puffer wie Walpole-Puffer (Essigsäure/NaAcetat, pH 3,6-5,6), Gomori-Aconitat-Puffer (Aconitinsäure/NaOH, pH 2,5-5,7), Kolthoff-Puffer (Borax/Succinat, pH 3,0-5,8), Sørensencitrat-Puffer (Dinatriumcitrat/HCl, pH 2,2-4,8) Sørensenglycin I-Puffer (Glycin, NaCl/HCl, pH 1,2-3,6), Clark und Lub-Phthalat I-Puffer (Kaliumbiphthalat/HCl, pH 2,2-3,8), Clark und Lub-Phthalat II-Puffer (Kaliumbiphthalat/NaOH, pH 4,0-6,2), Smith und Smith-Piperazin-Puffer (Piperacin, HCl/NaOH, pH 4,8-7,0), Clark und Lub-Kaliumchlorid/HCl-Puffer (KC1/HC1, pH 1,0-2,2), Gomori-Trismaleat-Puffer (Trismaleat/NaOH pH 5,2-8,6) oder Gomori-Succinat-Puffer (Succinat/NaOH, pH 3,8-6,0) in Frage. Auch Puffer wie MES, ADA, PIPES oder ACES, die in der Biochemie übliche Puffer sind, oder Aminosäurepuffer sind geeignete Puffer. Bevorzugt werden Puffer, die sich vorteilhafterweise aus schwachen Säuren und ihren Salzen herstellen lassen, wie z.B. Natriumacetat/Essigsäure, Natriumborat/Borsäure, Natriumphosphat/Phosphorsäure, Hydrogencarbonat/Soda, Natriumhydroxid/Citronensäure, Natriumhydroxid/Weinsäure. Auch Puffer aus schwachen Basen und ihren Salzen sind geeignet. Es können einzelne Puffer oder Mischungen zur Einstellung des pH-Wertes in den Dispersionen verwendet werden. Als Puffer bei freien basengrupi pentragenden Copolymeren wie zum Beispiel bei Copolymeren mit freien N-Alkyl-, Amino- oder Iminogruppen wird mindestens ein Puffersystem verwendet. Bevorzugt wird dabei der Pufferbereich des Puffersystems zwischen pH 7 bis 13 gewählt. Als Puffer bzw. Pufferlösungen mit einem Pufferbereich im basischen Milieu zwischen pH 7 bis 13 kommen beispielsweise Puffer wie Clark und Lub-Boratpuffer (Borsäure, KCl/NaOH, pH 7,8-10,0), Delory und King-Puffer (Carbonat/Bicarbonat, pH 9,2-10,7) oder Sørensen-Glycin II-Puffer (Glycin, NaCl/HCl, pH 8,4-13) in Frage. Auch Puffer wie Cholaminchlorid, BES, TES, HEPES, Acetamidoglycin, Glycinamid, Tris, Bicine, Tricine oder Glycylglycin, die in der Biochemie übliche Puffer sind, oder Aminosäurepuffer sind geeignete Puffer.

Bevorzugt werden die obengenannten Puffer, die sich vorteilhafterweise aus schwachen Säuren und ihren Salzen herstellen lassen. Auch Puffer aus schwachen Basen und ihren Salzen sind geeignet. Es können einzelne Puffer oder Mischungen zur Einstellung des pH-Wertes in den Dispersionen verwendet werden.

Werden polymere Puffer bzw. Pufferlösungen zur Neutralisation der freien Säure- oder Basengruppen im Copolymer verwendet, so lassen sich diese polymeren Puffer vorteilhafterweise durch Zugabe eines Neturalisationsmittels zu einem freien säuren- oder basengruppentragenden Polymers herstellen. Als Polymere geeignet sind durch radikalisch initiierte Polymerisation hergestellte freie säure- oder basengruppentragende Copolymere der (Meth)acrylsäure, ihrer Derivate, der Maleinsäure, der Fumarsäure, der Itaconsäure, der Crotonsäure, der Vinylsulfonsäure, der Vinylphosphonsäure des Polyethylenimins und/oder deren Salze, deren Ester sowie gegebenenfalls deren Anhydride. Als Anhydride kommen beispielsweise Acrylsäureanhydrid, Methacrylsäureanhydrid oder Maleinsäureanhydrid in Betracht. Als Polymere zur Herstellung polymerer Puffer kommen aber auch natürliche Polymere mit freien Säure- oder Basengruppen wie beispielsweise Alginsäure oder Hyaluronsäure in Frage. Die Monomerzusammensetzung der Dispersion und der zugese,tzten polymeren Pufferlösung kann gleich oder verschieden sein. Bevorzugt wird der polymere Puffer bzw. die polymere Pufferlösung aus einer Teilmenge der zu trocknenden Dispersion durch Zugabe eines Neutralisationsmittels hergestellt. Bei der Herstellung der Polymerlösung kann die Neutralisation der Monomereinheiten teilweise oder vollständig erfolgen. Vorteilhaft liegen 50 bis 100 Gew.-% der zur Salzbildung befähigten Monomere des Pufferpolymers in der Salzform vor. Bevorzugt sind 70 bis 95 Gew.-% der Monomere in der Salzform. Ganz besonders bevorzugt ist eine vollständige Neutralisation. Je nachdem welche freie Gruppen das Polymer trägt, werden dabei Basen oder Säuren zur Neutralisation verwendet. Als Neutralisationsmittel sind alle physiologisch akzeptablen Basen oder Säuren wie beispielsweise Natriumhydroxid, Natriumacetat, Natriumphosphat, Soda, Citronensäure, Weinsäure, Phosphorsäure, Essigsäure und/oder Ameisensäure einsetzbar. Aber auch die oben genannten Puffer- bzw. Pufferlösungen sind geeignet.

Es können einzelne Säuren, Basen oder Puffer zur Neutralisation oder deren Kombinationen zur Herstellung der polymeren Pufferlösung verwendet werden.

Die Ausgangsdispersion kann so einfach über die Einstellung eines sauren oder basischen pH's durch Zugabe mindestens eines Puffersystems in eine Dispersion überführt werden, die nach Trocknung ein redispergierbares Pulver ergibt. Als Puffersysteme sind einzelne Puffer und/oder polymere Puffer oder Mischungen von Puffern und/oder polymeren Puffern geeignet.

Unabhängig von der Natur der salzbildenden Gruppe kann immer der gleiche Puffer und/oder polymere Puffer verwendet werden. Es bedarf dabei nicht des Zusatzes einer zusätzlichen Säure oder Base, obwohl dieses durchaus möglich wäre. Ebenso ist es möglich, die ohne Pufferzusatz getrockneten durch radikalisch initiierte Polymerisation erhaltenen Dispersionspulver durch Einrühren in mindestens einen Puffer und/oder mindestens einen polymeren Puffer zu redispergieren.

Aus ökonomischen Gründen beträgt der Feststoffgehalt der zu trocknenden Dispersion in der Regel mehr als 25 Gew.-%. Bei der Herstellung der Polymerpufferlösung, ausgehend von einer solchen Dispersion, ist es jedoch zweckmäßig, zunächst einen Feststoffgehalt kleiner 15 Gew.-% einzustellen und anschließend mit einem Neutralisierungsmittel zu versetzen.

Die auf diese Weise hergestellte verdünnte Polymerpufferlösung wird anschließend mit der zu trocknenden Dispersion vereint.

Der Feststoffgehalt der Kombination aus Dispersion und Puffer und/oder Polymerpufferlösung liegt zwischen 1 und 50 Gew.-%. Bevorzugt sind 5 bis 40 Gew.-%, besonders bevorzugt sind 20 bis 35 Gew.-%.

Es ist von Vorteil, daß die über den erfindungsgemäßen Puffer- und/oder polymeren Pufferzusatz hergestellten Dispersionen pH-Schwankungen bei der Verarbeitung verkraften, ohne daß es zu Koagulatbildung kommt. Durch den Pufferzusatz läßt sich der Öffnungs-pH eines Filmbildners, d.h. der pH-Wert, ab dem ein Polymer zu quellen und schließlich sich aufzulösen beginnt in präzisen Bereichen variieren. Dies ist insbesondere von Vorteil, wenn eine gezielte Freisetzung eines Wirkstoffes gewünscht wird und so eine optimale Bioverfügbarkeit erzielt werden soll.

Zur Herstellung der erfindungsgemäßen redispergierbaren Polymerpulver eignen sich prinzipiell alle durch radikalische initiierte Polymerisation erhältlichen Copolymere.

Als Monomerbausteine dieser Copolymere können monoethylenisch ungesättigte C₃-C₈-Mono- und Dicarbonsäuren, ihre Anhydride, ihre Ester, ihre Amide, ihre Salze oder Mischungen aus den genannten Carbonsäuren, Anhydriden, Estern, Amiden und Salzen verwendet werden.

Geeignete Carbonsäuren sind beispielsweise Acrylsäure, Methacrylsäure, Maleinsäure, Fumarsäure, Itaconsäure oder Crotonsäure. Als Anhydride kommen beispielsweise Acrylsäureanhydrid, Methacrylsäureanhydrid oder Maleinsäureanhydrid in Betracht.

Als Ester sind beispielsweise die C₁-C₁₀-Alkylester oder C₁-C₁₀-Hydroxyalkylester der oben genannten Carbonsäuren geeignet. Bevorzugt geeignete Alkylester sind die Methyl-, Ethyl-, Propyl-, Butyl-, i-Butyl-, tert.-Butyl-, Pentyl-, i-Pentyl-2,2-Dimethylpropyl-, Hexyl-, i-Hexyl-, Heptyl-, i-Heptyl-, Octyl-, i-Octyl-, 2-Ethylhexyl-, Nonyl-, i-Nonyl-, Decyl- oder i-Decylester der Acryl- und/oder Methacrylsäure. Besonders bevorzugt werden die Methyl-, Ethyl-, Propyl-, Butyl-, i-Butyl oder tert.-Butylester.

Werden die Monomere in Form ihrer Salze zur Polymerisation verwendet, so sind die Erdalkali-, Alkali- oder Ammoniumsalze oder die Salze organischer Amine bevorzugt, besonders bevorzugt sind die Alkali- oder Ammoniumsalze.

Als basische Monomerbausteine der Copolymere sind Vinylimidazol, Vinylimidazolin, Vinylimidazolidin, Vinylpyridin, Monoalkyl- oder Dialkylaminoalkylester oder Monoalkyl- oder Dialkylaminoalkylamide der oben genannten ungesättigten Carbonsäuren geeignet.

Es können einzelne Monomere oder Mischungen als Ausgangsmonomere für die Herstellung der Copolymere verwendet werden.

Für die erfindungsgemäßen redispergierbaren Dispersionspulver eignen sich vorteilhafterweise Copolymere, die 80 bis 15 Gew.-%, bevorzugt 70 bis 30 Gew.-% eines zur Salzbildung befähigten radikalisch polymerisierbaren Monomeres enthalten.

Bevorzugt enthalten die Dispersionspulver als weitere Monomerkomponente mit 20 bis 85 Gew.-% Einheiten, bevorzugt 30 bis 70 Gew.-%, wenigstens einen der oben genannten Alkylester der Acryl- und/oder Methacrylsäure.

Als Polymere eignen sich vorteilhafterweise Copolymere aus Methacrylsäure und Ethylacrylat, Methacrylsäure und Methacrylsäureester, Terpolymere aus Dimethylaminoethylmethacrylat, Methacrylsäuremethyl- und -butylestern sowie Copolymere aus Vinylacetat und Crotonsäure. Bevorzugt sind Copolymere aus Methacrylsäure und Ethylacrylat.

Weiterhin kann die Polymerlösung bzw. Dispersion weitere Hilfsstoffe enthalten.

Als Hilfsstoffe kommen Polysaccharide wie Cellulosen z.B. Hydroxyalkylcellulose wie Methylcellulose, Ethylcellulose oder Hydroxy(alkyl)alkylcellulosen wie Hydroxypropylcellulose oder Hydroxypropylmethylcellulosephthalat, Stärke, abgebaute Stärke, Pektin, Chitin, Chitosan, Arabinogalaktan, Xylan, Xanthan, Galaktomannane wie Ghattigummi, Johannesbrotkernmehl oder Tragant in Betracht.

Aber auch Proteine wie Casein oder Gelatine oder Schellack können als Hilfsstoffe verwendet werden.

Weiterhin eignen sich Zucker wie Mono-, Di- und Trisaccharide beispielsweise Glucose, Mannose oder Saccharose, Zuckerderivate, Zuckeralkohole oder Harnstoff. Unter Hilfsstoffen sind auch Substanzen wie beispielsweise Pentaerythrit, Pentaerythrittetraacetat, Polymere wie z.B. Polyethylen- bzw. Polypropylenoxide und deren Blockcopolymere, Phosphatide wie Lecithin, Lysolecithin, Homo- und Copolymere des Vinylpyrrolidons, Tenside, Citronensäure, Bernsteinsäure, Gallensäuren, Sterine und weitere Stoffe, wie Ford, J.L. (Pharm. Acta Helv. 61, 69-88, 1986) zu entnehmen ist, zu verstehen.

Unter Tensiden sind beispielsweise Substanzen wie die verschiedenen Brij®-Typen, d.h. Cetyl-, Lauryl-, Oleyl- oder Stearylether mit 2 bis 100 Polyoxyethyleneinheiten, die verschiedenen Myrj®-Typen Stearinester mit 8 bis 100 Polyoxyethyleneinheiten (=POE), die verschiedenen Span®-Typen wie Span 20 (Sorbitanmonolaurate), Span 40 (Sorbitanmonopalmitate), Span 60 (Sorbitanmonostearate) oder Span 80 (Sorbitanmonooleate), die verschiedenen Tween®-Typen wie Tween 20 (POE (20)-Sorbitanmonolaurat), Tween 40 (POE (20)-Sorbitanmonopalmitate), Tween 60 (POE (20)-Sorbitanmonostearate) oder Tween 80 (POE (20)-Sorbitanmonoleate), die verschiedenen Triton®-Typen (Octylphenolethoxylate) wie Triton X-15, X-35, X-100 CG, X-305 (70 %), X-405 (70 %), X-705 (70 %), die ethoxylierten Ricinusöle wie Polyoxyethylenglyceroltriricinoleat 35 (Cremophor® EL) oder Polyoxyethylenglyceroltrihydroxystearat 40 (Cremophor® RH40), die ethoxylierten 12-Hydroxystearinsäure wie Polyoxyethylen-660-12'-hydroxystearat (Solutol® HS15) oder Natriumlaurylsulfat zu verstehen.

Weitere Hilfsstoffe können beispielsweise Füllstoffe, Glättmittel, Glanzmittel, Netzmittel, Schmiermittel, Formentrennmittel, Weichmacher, Treibmittel, Stabilisatoren, Emulgatoren, Farbstoffe, Geschmackstoffe, Steckmittel, Fließmittel sowie deren Mischungen sein.

Beispiele für Weichmacher beinhalten niedermolekulare Poly-(alkylenoxide), wie z.B. Poly(ethylenglycole), Poly(propylenglycole), Poly(ethylenpropylenglycole), organische Weichmacher mit niederem Molekulargewicht wie Glycerin, Pentaerythrit, Glycerinmonoacetat, -diacetat oder -triacetat, Propylenglycol oder Natriumdiethylsulfosuccinat, Phthalsäureester, Citronensäureester, Sebacinsäureester oder acetylierte Fettsäureglyceride.

Beispiele für Farbstoffe sind bekannte Azofarbstoffe, organische oder anorganische Pigmente oder Farbmittel natürlicher Herkunft. Anorganische Pigmente sind bevorzugt.

Darüberhinaus können noch andere Additive zugefügt werden, wie tierische oder pflanzliche Fette, bevorzugt in ihrer hydrierten Form, besonders solche, die bei Raumtemperatur (20°C) fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Die gleiche Funktion können auch Wachse wie Carnaubawachs, Candelillawachs, Ouricurriwachs, Zuckerrohrwachs, Retamowachs, tierische Wachse wie Bienenwachs, Schellackwachse, Insektenwachse oder Wollwachse, Erdöl-, Braunkohlen-, Torf- und andere Montanwachse, Polyolefinwachse, Paraffinwachse, Säurewachse, Esterwachse, Alkohol- oder Amidwachse erfüllen. Die Wachse können naturbelassen, modifiziert, teil- oder vollsynthetisch sein. Selbstverständlich können auch einzelne Wachskomponenten wie z.B. Wachssäuren, Wachsalkohole, Wachsketone, Hydroxywachssäuren, Paraffine, Harzsäuren, Polyterpene, Harzalkohole, Sterine, Vaseline oder Fettsäuren oder Mischungen beigefügt werden.

Diese Fette, Wachse, Fett- oder Wachsderivate können vorteilhaft alleine oder zusammen mit Mono- und/oder Diglyceriden oder Phosphatiden, besonders Lecithin, beigemischt werden. Die Mono- und Diglyceride stammen vorzugsweise von den oben beschriebenen Fetttypen ab, d.h. C₁₂-, C₁₄-, C₁₆- und C₁₈-Fettsäuren.

Als Hilfsstoffe eignen sich fernerhin die verschiedensten Emulgatoren. Handelsübliche Emulgatoren sind beispielsweise in M. und I. Ash, Handbook of Industrial Surfactans, Gower Publishing Co., Hants, 1993 aufgeführt. Sie können niedermolekulare oder polymere Verbindungen sein. Niedermolekulare Verbindungen enthalten im allgemeinen einen geradkettig oder verzweigten, gesättigten oder ungesättigten oder ungesättigten, cyclischen oder acyclischen, aromatischen oder aliphatischen Alkylrest mit 8 bis 40, bevorzugt 10 bis 30, ganz besonders bevorzugt 12 bis 22 Kohlenstoffatomen im Molekül.

Ferner können auch Stabilisatoren zugefügt werden, wie z.B. Antioxidantien, Lichtstabilisatoren, Hydroperoxidvernichter, Radikalfänger und Stabilisatoren gegen mikrobiellen Befall.

Als Hilfsstoffe gelten auch Zusätze von Basen oder Säuren zur Steuerung der Löslichkeit eines Wirkstoffes (siehe Z.B.K. Thoma et al., Pharm. Ind. 51, 1989: 98-101).

Die Hilfsstoffe können der Dispersion vor oder nach der Trocknung zugesetzt werden.

Die Hilfsstoffe werden der Dispersion gegebenenfalls in einer Menge von 0,5 bis 50 Gew.-%, bevorzugt 1 bis 30 Gew.-% bezogen auf den Gesamtfeststoffgehalt der Dispersion zugesetzt.

Freie säuren- oder basengruppenhaltige Polymere lassen sich in bekannter Weise durch radikalisch initiierte Polymerisation nach allen bekannten Verfahren herstellen.

Die vorteilhaft zur Herstellung der redispergierbaren Polymerpulvern eingesetzten Copolymere können nach verschiedenen Verfahren wie beispielsweise durch Fällungspolymerisation, umgekehrte Emulsionspolymerisation, umgekehrte Suspensionspolymerisation, Emulsionspolymerisation, Suspensionspolymerisation oder Lösungspolymerisation hergestellt werden.

Die Polymerisation kann diskontinuierlich oder kontinuierlich, mit oder ohne Verwendung von Saatpolymeren, unter Vorlage aller oder einzelner Bestandteile des Reaktionsgemisches, oder unter teilweiser Vorlage und Nachdosierung der oder einzelner Bestandteile des Reaktionsgemisches, oder nach dem Dosierverfahren ohne Vorlage durchgeführt werden.

In DE-A-4 325 158 wird zum Beispiel ein Verfahren zur Fällungspolymerisation beschrieben.

Für die Trocknung der fertigen Dispersion kommen alle üblichen Technologien wie beispielsweise die Dünnschichttrocknung, die Sprühwirbelschichttrocknung, die Sprühtrocknung oder die Gefriertrocknung in Frage.

Die zu zerstäubenden Dispersionen können auch Sprühhilfsmittel wie Polyvinylalkohole, Cellulosederivate, Stärkederivate, Ligninsulfonate, Polyacrylsäure und Polyacrylamide enthalten. Als Antiblockmittel können z.B. Aluminium-Silikate, wie Bentonit, Kieselgur, kolloides Silicagel, Diatomeenerde, Calciumcarbonat, Magnesiumsilikate wie Talkum oder Tricalciumphosphat zugesetzt werden.

Die Zerstäubung erfolgt vorzugsweise als hydrodynamische Zerstäubung durch Flüssigkeitsdruck oder Luftdruck über Düsen wie beispielsweise Einstoff-, Mehrstoff- oder Scheiben-Düsen mit Hilfe herkömmlicher Zerstäubungsvorrichtungen.

Das Grundprinzip ist die Zerstäubung der Lösung in sehr kleine Tröpfchen. Diese Tröpfchen haben eine sehr große Oberfläche, was eine schnelle Verdampfung gestattet. Der erforderliche geringe Durchmesser der Tröpfchen wird durch die Geschwindigkeit des Zerstäuberrades oder den Druck des Zerstäubergases erzielt. Der erreichbare Verdampfungsgrad genügt, um die Feuchtigkeit völlig aus den Tröpfchen zu entfernen. Durch die Verdampfungskälte und die Kürze der Schwebezeit wird eine Wärmebeschädigung des Produktes vermieden.

Die Trocknung der Flüssigkeitströpfchen kann in einem Sprühturm herkömmlicher Bauart erfolgen, wobei Luft oder ein inertes Gas wie Stickstoff, Argon oder Helium als Trocknungsgas verwendet wird, welches im Gleichstrom oder Gegenstrom mit den Flüssigkeitstropfen durch den Trocknungsturm geleitet werden kann. Vorzugsweise wird das Trocknungsgas im Gleichstrom eingesetzt, wobei die Turmeingangstemperatur des Gases bei 60 bis 160, vorzugsweise 90 bis 140°C liegt und die Turmausgangstemperatur bei 40 bis 100, vorzugsweise 60 bis 80°C. Die Verdampfung des Lösungsmittels kann sowohl bei Normaldruck als auch bei 0,6 bis 1,2 bar erfolgen.

Das entstehende Pulver kann in üblicher Weise über ein Zyklon von dem Gasstrom getrennt werden.

Der Restlösungsmittelgehalt des so entstehenden Pulvers beträgt im allgemeinen nicht über 7,5 Gew.-%. Die Teilchengrößen der entstehenden Pulverteilchen liegen im allgemeinen bei 10 bis 150 µm. Bei einer Sprühgranulation können Teilchengrößen bis 450 µm erreicht werden.

Gegenüber dem Stand der Technik lassen sich die erfindungsgemäßen in wäßriger Lösung redispergierbaren Polymerpulver erheblich besser redispergieren. Die Redispergierung lag in allen Versuchen > 90 %. Von Vorteil ist, daß sich Filme aus den erfindungsgemäßen Dispersionen schneller auflösen als Filme aus Ausgangsdispersionen. Überzogene Darreichungsformen können daher im Dünndarm den Wirkstoff rascher freisetzen.

Die erfindungsgemäßen redispergierbaren Dispersionspulver bzw. die daraus hergestellten Dispersionen oder andere Folgeprodukte eignen sich für alle festen oder halbfesten kosmetischen oder pharmazeutischen Zubereitungen oder für Zubereitungen im Pflanzenschutz oder Tierschutz.

Unter festen oder halbfesten kosmetischen oder pharmazeutischen Zubereitungen sind z.B. Tabletten, Wirkstoffkristalle, Mirkotabletten, Dragees, Pellets, Pastillen, Kapseln, Mikrokapseln und Granulate zu verstehen, ohne auf diese Zubereitungen beschränkt zu sein.

Eine besondere Anwendungsmöglichkeit der redispergierbaren Dispersionspulver ist die Herstellung von transdermalen therapeutischen Systemen.

Unter Zubereitungen im Pflanzenschutz sind beispielsweise Pheromonfallen für Schadinsekten, bei denen eine gezielte, langanhaltende Pheromonfreisetzung erforderlich ist, zu verstehen.

### Beispiele:

### Beispiel 1

In den Beispielen 1, 2, 5 und 6 verwendete Puffer
Merck Puffer pH 5,0 enthält auf 1 l
20,256 g Citronensäure
7,84 g NaOH

Merck Puffer pH 6,0 enthält auf 1 l
12,526 g Citronensäure
6,32 g NaOH

Merck Puffer pH 4,66 enthält auf 1 l
6,005 g Essigsäure
8,204 g Na-Acetat
600 g einer 30 %igen Eudragit® L30D Dispersion werden mit 100 g eines 0,9 %igen Citronensäurepuffers unter Rühren versetzt. Der resultierende pH-Wert beträgt anschließend 4,0. Die gesamte Lösung wird anschließend nach bekannten Methoden sprühgetrocknet (Trocknungsgas: Stickstoff-Gleichstrom, Eingangstemperatur: 125°C, Ausgangstemperatur: 50°C). Man erhält ein redispergierbares Polymerpulver, welches sich durch Einrühren in Wasser redispergieren läßt. Der Koagulatanteil, gemessen < 50 µm beträgt 0 % (Messung s. Beispiel 7). Der Öffnungs-pH beträgt 5,5.

### Beispiel 2

180 g eines Methacrylsäure/Ethylacrylat-Copolymeren (Verhältnis 1:1) werden in eine wäßrige Lösung mit einem 0,8 %igem Citratpuffer eingerührt. Man erhält so eine Dispersion des zuvor nicht redispergierbaren Methacrylsäurecopolymeren.

### Beispiel 3

Herstellung eines redispergierbaren Pulvers aus Eudragit® L30D (Röhm GmbH, Acrylatdispersion aus Ethylacrylat und Methacrylsäure)
a) Herstellung einer wäßrigen Polyacrylatpufferlösung
   93,4 g einer 10 %igen Dispersion (Eudragit® L30D) wird durch Zugabe von 20,7 g einer wäßrigen 10 %igen NaOH-Lösung in 114,1 g einer wäßrigen Acrylatlösung mit einem pH-Wert von 7,0 überführt.
b) Herstellung der Mischung
   1000 g einer 30 %ige Dispersion (Eudragit® L30D, pH ca. 2-3) werden mit 114 g der unter a) hergestellten Lösung versetzt. Es resultiert eine Polyacrylat-Dispersion mit einem pH-Wert 5,2. Die Dispersion wird nachfolgend sprühgetrocknet. Man erhält ein redispergierbares Pulver.

### Beispiel 4

a) Herstellung einer wäßrigen Polyacrylatpufferlösung
   93,4 g einer 10 %igen Dispersion (Kollicoat® MAE 30D, BASF AG, Acrylatdispersion aus Ethylacrylat und Methacryli säure, Feststoffgehalt ca. 30 %) wird durch Zugabe von 20,7 g einer wäßrigen 10 %igen NaOH-Lösung in 114,1 g einer wäßrigen Acrylatlösung mit einem pH-Wert von 7,0 überführt.
b) Herstellung der Mischung
   1000 g einer 30 %igen Dispersion (Kollicoat® MAE 30D, BASF AG, Acrylatdispersion aus Ethylacrylat und Methacrylsäure, Feststoffgehalt ca. 30 %, pH ca. 2-3) werden mit 114 g der unter a) hergestellten Lösung versetzt. Es resultiert eine Polyacrylat-Dispersion mit dem pH-Wert 5,2. Die Dispersion wird nachfolgend sprühgetrocknet. Man erhält ein redispergierbares Pulver.

### Beispiel 5

### Tablettenüberzug

In einem Horizontaltrommelcoater (Typ Accela Cota, Fa. Manesty) wurden 5000 g Propanolol-Tabletten mit einem Wirkstoffgehalt von 40 mg und einem Tablettengewicht von 250 mg mit einem magensaftresistenten Filmlack auf Basis eines redispergierbaren Methacrylsäure-Ethylacrylat-Copolymeren mit 2 % Acetatpuffer pH 4,66 bezogen auf den Filmbildner überzogen, wobei 6 mg/cm² magensaftresistenter Filmbildner bzw. 8 mg/cm² Feststoff aufgetragen wurden.

Die aufgesprühte Dispersion wies folgende Zusammensetzung auf:

| | | |
|---|---|---|
| Pigmentsuspension | Titandioxid | 0,5 % |
| | Talkum | 2,0 % |
| | Sicovit Rot 30 | 0,5 % |
| | Kollidon 30 | 0,5 % |
| | Wasser | 10,0 % |
| Lacksuspension | Methacrylsäure-Ethylacrylat-Copolymer mit 2 % Acetatpuffer pH 4,66 | 15,0 % |
| | Triethylcitrat | 1,5 % |
| | Wasser | 70,0 % |
| | Gesamtgewicht | 100,0 % |

Die Homogenisierung der Pigmentsuspension erfolgte in einer Korundscheibenmühle. Zur Redispergierung des Methylacrylsäure-Ethylacrylat-Copolymeren mit 2 % Acetatpuffer wurde der Feststoff langsam mit einem Flügelrührer in Wasser eingerührt, wobei sich sofort eine sehr feine Dispersion ausbildete.

Von der Sprühsuspension wurden 1680,8 g bei einer Zulufttemperatur von 46°C, einer Abblufttemperatur von 33°C und einer Sprührate von 40 g/min aufgesprüht. Der Überzug war sehr glatt, gleichmäßig und einheitlich rot gefärbt.

Die Freisetzung in künstlichem Magensaft (pH: 1,2) zeigte, daß der Überzug über 2 h magensaftresistent war und die anschließende Umpufferung auf pH 6,8 belegte die schnelle Öffnung des Überzuges im Neutralbereich (99 % Freisetzung innerhalb 60 min).

| Freisetzungswerte: | | | |
|---|---|---|---|
| 60 min | pH 1,2 | 0,5 % | freigesetzter Wirkstoff |
| 120 min | pH 1,2 | 0,8 % | freigesetzter Wirkstoff |
| Umpufferung auf pH 6,8 mit Phosphatpuffer | | | |
| 150 min | 99,0 % | | freigesetzter Wirkstoff |

### Beispiel 6

### Redispergierbarkeit

20 g redispergierbares Methacrylsäure-Ethylacrylat-Copolymer mit 2 % Acetatpuffer bzw. 5 % Citratpuffer jeweils bezogen auf den Filmbildner wurden in 80 g demineralisiertem Wasser mittels eines Magnetrührers suspendiert. Die gesamte Rührzeit betrug 60 min. Anschließend wurde die Suspension durch zwei übereinanderliegende Siebe mit den Maschenweiten 125 µm und 50 µm gegeben und der Rückstand - sofern vorhanden - mit wenig Wasser gewaschen. Nach Trocknung wurde der Rückstand gravimetrisch bestimmt.

| Ergebnis | Rückstand | |
|---|---|---|
| | 125 µm | 50 µm |
| Methacrylsäure-Ethylacrylat-Copolymer mit 2 % Acetatpuffer pH 4,66 | 0,00 % | 0,02 % |
| Methacrylsäure-Ethylacrylat-Copolymer mit 5 % Citratpuffer pH 6,0 | 0,00% | 0,01 % |

## Patentansprüche

1. Verfahren zur Herstellung von in wäßriger Lösung redispergierbaren Polymerpulvern, enthaltend freie säure- oder basengruppentragende Copolymere und ggf. Hilfsstoffe, dadurch gekennzeichnet, daß man durch die Einstellung des pH-Wertes einen Teil der freien Säure- oder Basengruppen der Copolymere in ihr Salz überführt und man den pH-Wert der Dispersion durch Zugabe mindestens eines Puffersystems auf pH 2,0 bis 6,5 im Falle säuregruppentragender Copolymere bzw. pH 7,5 bis 12 im Falle basengruppentragender Copolymere vor der Trocknung einstellt.

2. Verfahren nach Anspruch 1, dadurch gekennzeichnet, daß man den pH-Wert der Dispersion durch Zugabe mindestens eines Puffersystems auf pH 3,0 bis 6,0 im Falle säuregruppentragender Copolymere bzw. 7,5 bis 11 im Falle basengruppentragender Copolymere vor der Trockrung einstellt.

3. Verfahren nach Anspruch 1 oder 2, dadurch gekennzeichnet, daß als Puffersystem Salze aus schwachen Säuren und starken Basen bzw. Salze aus starken Säuren und schwachen Basen verwendet werden.

4. Verfahren nach Anspruch 1 oder 2, dadurch gekennzeichnet, daß als Puffersystem polymere Puffer, erhältlich durch Zugabe eines Neutralisationsmittels zu einem freien säure- oder basengruppentragenden Polymers, verwendet werden.

5. Verfahren nach einem der Ansprüche 1 bis 4, dadurch gekennzeichnet, daß man als Polymerpulver freie carboxylgruppen- oder aminogruppentragende Copolymere verwendet.

6. Verfahren nach einem der Ansprüche 1 bis 5, dadurch gekennzeichnet, daß man als Polymerpulver Copolymere mit 20 bis 85 Gew.-% eines Alkylesters der Acryl- und/oder Methacrylsäure und 80 bis 15 Gew.-% eines zur Salzbildung befähigten radikalisch polymerisierbaren Monomeren verwendet.

7. In wäßriger Lösung redispergierbare Polymerpulver erhältlich nach einem Verfahren gemäß den Ansprüchen 1 bis 6.

8. Verwendung von in wäßriger Lösung redispergierbaren Polymerpulvern gemäß dem Anspruch 7 in kosmetischen oder pharmazeutischen Zubereitungen oder Zubereitungen für den Pflanzenschutz.

9. Verwendung eines Puffersystems gemäß den Ansprüchen 3 oder 4 zum Redispergieren ohne Pufferzusatz getrockneter Polymerpulver enthaltend freie säuregruppen- oder basengruppentragende Copolymere, wobei man für säuregruppentragende Copolymere ein Puffersystem, mit dem sich der pH-Wert der Dispersion auf 2,0 bis 6,5 einstellen läßt, und für basengruppentragende Copolymere ein Puffersystem, mit dem sich der pH-Wert der Dispersion auf 7,5 bis 12 einstellen läßt, verwendet und man durch diese Einstellung des pH-Wertes einen i Teil der freien Säure- oder Basengruppen der Copolymere in ihr Salz überführt.

10. Kosmetische oder pharmazeutische Zubereitungen oder Zubereitungen für den Pflanzenschutz enthaltend in wäßriger Lösung redispergierbare Polymerpulver gemäß dem Anspruch 7 und übliche Zusatzstoffe.

## Claims

1. A process for preparing polymer powders redispersible in aqueous solution comprising copolymers carrying free acid or base groups and, if appropriate, auxiliaries, which comprises converting some of the free acid or base groups of the copolymers into their salt by adjusting the pH and adjusting the pH of the dispersion by the addition of at least one buffer system to a pH of from 2.0 to 6.5 in the case of copolymers carrying acid groups or to a pH of from 7.5 to 12 in the case of copolymers carrying base groups prior to drying.

2. A process as claimed in claim 1, wherein the pH of the dispersion is adjusted by the addition of at least one buffer system to a pH of from 3.0 to 6.0 in the case of copolymers carrying acid groups or to from 7.5 to 11 in the case of copolymers carrying base groups prior to drying.

3. A process as claimed in claim 1 or 2, wherein the buffer system used comprises salts of weak acids and strong bases or salts of strong acids and weak bases.

4. A process as claimed in claim 1 or 2, wherein the buffer system used comprises polymeric buffers obtainable by adding a neutralizing agent to a polymer carrying free acid or base groups.

5. A process as claimed in any of claims 1 to 4, wherein the polymer powder used comprises copolymers carrying free carboxyl or amino groups.

6. A process as claimed in any of claims 1 to 5, wherein the polymer powder used comprises copolymers having from 20 to 85% by weight of an alkyl ester of acrylic and/or methacrylic acid and from 80 to 15% by weight of a free-radically polymerizable monomer capable of forming salts.

7. Polymer powders redispersible in aqueous solution obtainable by any of the processes as claimed in claims 1 to 6.

8. The use of polymer powders redispersible in aqueous solution as claimed in claim 7 in cosmetic or pharmaceutical formulations or compositions for crop protection.

9. The use of a buffer system as claimed in claims 3 or 4 for redispersing polymer powders comprising copolymers carrying free acid groups or base groups dried without the addition of buffer, which comprises using, in the case of copolymers carrying acid groups, a buffer system with which the pH of the dispersion can be adjusted to from 2.0 to 6.5 and, in the case of copolymers carrying base groups, a buffer system with which the pH of the dispersion can be adjusted to from 7.5 to 12 and converting some of the free acid or base groups of the copolymers into their salt using this adjustment of the pH.

10. Cosmetic, pharmaceutical or crop protection formulations comprising polymer powders redispersible in aqueous solution as claimed in claim 7 and customary additives.

## Revendications

1. Procédé de préparation de poudres polymères redispersables en solution aqueuse, contenant des copolymères à groupes acides ou basiques libres et le cas échéant des produits auxiliaires, caractérisé par le fait que l'on convertit une partie des groupes acides ou basiques libres des copolymères en leurs sels par réglage du pH et on règle le pH de la dispersion, par addition d'au moins un système tampon, à un niveau de 2 ,0 à 6,5 dans le cas de copolymères à groupes acides et à un niveau de 7,5 à 12 dans le cas de copolymères à groupes basiques, avant le séchage.

2. Procédé selon la revendication 1, caractérisé par le fait que l'on règle le pH de la dispersion par addition d'au moins un système tampon à un niveau de 3,0 à 6,0 dans le cas de copolymères à groupes acides et de 7,5 à 11 dans le cas de copolymères à groupes basiques, avant séchage.

3. Procédé selon la revendication 1 ou 2, caractérisé par le fait que l'on utilise, en tant que systèmes tampon, des sels d'acides faibles et de bases fortes ou respectivement des sels d'acides forts et de bases faibles.

4. Procédé selon la revendication 1 ou 2, caractérisé par le fait que l'on utilise, en tant que systèmes tampons, des tampons polymères obtenus par addition d'un agent neutralisant à un polymère à groupes acides ou basiques libres.

5. Procédé selon une des revendications 1 à 4, caractérisé par le fait que l'on utilise, en tant que poudres polymères, des copolymères à groupes carboxyle libres ou à groupes amino libres.

6. Procédé selon une des revendications 1 à 5, caractérisé par le fait que l'on utilise, en tant que poudres polymères, des copolymères contenant 20 à 85% en poids d'un ester alkylique de l'acide acrylique et/ou méthacrylique et de 80 à 15% en poids d'un monomère polymérisable par polymérisation radicalaire et apte à former des sels.

7. Poudres polymères redispersables en solution aqueuse obtenues par un procédé selon les revendications 1 à 6.

8. Utilisation de poudres polymères redispersables en solution aqueuse selon la revendication 7 dans des compositions cosmétiques ou pharmaceutiques ou des compositions phytosanitaires.

9. Utilisation d'un système tampon selon les revendications 3 ou 4 pour redisperser des poudres polymères séchées sans addition d'un tampon, qui contiennent des copolymères à groupes acides libres ou à groupes basiques libres, dans laquelle, pour les copolymères à groupes acides, on utilise un système tampon permettant de régler le pH de la dispersion à un niveau de 2,0 à 6,5 et pour les copolymères à groupes basiques on utilise un système tampon permettant de régler le pH de la dispersion à un niveau de 7,5 à 12, et, par ce réglage du pH, on convertit une partie des groupes acides libres ou basiques libres des copolymères en leurs sels.

10. Compositions cosmétiques ou pharmaceutiques ou compositions phytosanitaires contenant des poudres polymères redispersables en solution aqueuse selon la revendication 7 et des additifs usuels.
